# EUROPEAN PATENT SPECIFICATION

(11) **EP 1 428 888 B1**
(45) Date of publication and mention of the grant of the patent: **22.10.2008**
(21) Application number: 02027877.6
(22) Date of filing: 13.12.2002
(51) Int. Cl.: C12P 7/62

(54) **Method for the production of (1S,4R)-(-)-4-Hydroxycyclopent-2-enyl esters**
Verfahren zur Herstellung von (1S,4R)-(-)-4-Hydroxycyclopent-2-enyl Estern
Procédé de préparation d'esters de (1S,4R)-(-)-4-Hydroxycyclopentényle

(43) Date of publication of application: 16.06.2004
(73) Proprietor: Archimica GmbH, 65929 Frankfurt am Main (DE)
(72) Inventor: Wisdom, Richard, Dr., 65817 Eppstein (DE)
(74) Representative: Schweitzer, Klaus

(56) References cited:
- DD-A- 290 663
- US-B1- 6 448 051
- NAEMURA K ET AL: "Enantioselective Acylation of Alcohols Catalyzed by Lipase QL from Alcaligenes sp.: A Predictive Active Site Model for Lipase QL to Identify the Faster Reacting Enantiomer of an Alcohol in this Acylation" TETRAHEDRON: ASYMMETRY, ELSEVIER SCIENCE PUBLISHERS, AMSTERDAM, NL, vol. 7, no. 6, 1 June 1996 (1996-06-01), pages 1581-1584, XP004047639 ISSN: 0957-4166
- NAEMURA K ET AL: "Enantioselective Acylation of Primary and Secondary Alcohols Catalyzed by Lipase QL from Alcaligenes sp.: A Predictive Active Site Model for Lipase QL to Identify which Enantiomer of an Alcohol Reacts Faster in this Acylation" TETRAHEDRON: ASYMMETRY, ELSEVIER SCIENCE PUBLISHERS, AMSTERDAM, NL, vol. 7, no. 11, 8 November 1996 (1996-11-08), pages 3285-3294, XP004068988 ISSN: 0957-4166
- GHORPADE S R ET AL: "Desymmetrization of meso-cyclopenten-cis-1,4-diol to 4-(R)-hydroxycyclopent-2-en-1-(S)-acetate by irreversible transesterification using ChirazymeR)" TETRAHEDRON: ASYMMETRY, ELSEVIER SCIENCE PUBLISHERS, AMSTERDAM, NL, vol. 10, no. 5, 12 March 1999 (1999-03-12), pages 891-899, XP004162512 ISSN: 0957-4166

## Description

The instant application refers to a new method for the production of (1S, 4R)-(-)-4-hydroxy cyclopent-2-enyl esters, preferably (1S, 4R)-(-)-4-hydroxy cyclopent-2-enyl acetate and -proprionate and its derivatives.

(1S, 4R)-(-)-4-Hydroxy cyclopent-2-enyl acetate has been identified as a very useful synthon in the preparation of prostaglandins and other cyclopentanoid products (M. Nara et al, Tetrahedron 36 (1980) 3161; M. Harre et al, Angew. Chemie 94 (1982) 496; Johnson CR. et al., J. Am. Chem. Soc, 115, 11014-11015, (1993)). As a result, numerous approaches have been described over the years to prepare this material. Many of these approaches utilise the selective ability of enzymes and, in particular esterases and lipases, to recognise and preferentially produce the preferred enantiomer.

Two related enzymatic routes to the required compound have been described. Firstly it is possible to utilise an enzyme to selectively hydrolyse cis-3,5-diacetoxy cyclopent-1-ene, which may readily be prepared by the method described by Deardorff (Deardorff et al., Tetrahedron Letters 26, (46), pp 5615-5618 (1985)). Laumen et al. (Laumen et al., Tetrahedron Letters, 25 (51), pp 5875-5878, (1984)) screened a number of enzymes to identify their selectivity. US-A- 4,618,690 describes an example with Pig liver esterase in which a yield of the required enantiomer of 86% on starting cis-3,5-diacetoxy cyclopent-1-ene is obtained, however the ee is only 66%. Although it was possible to increase ee to 86% on one crystalisation and thereafter probably higher on subsequent recrystalisations, this would significantly reduce yields. It is known that commercial preparations of pig liver esterases are a mixture of variants, and it may be that use of a single cloned and expressed variant may give higher selectivity. A recent patent, US-B1-6,448,051 identifies a range of organisms of Trichosporon sp which show high selectivity in their hydrolysis of cis-3,5-diacetoxy cyclopent-1-ene and which produce the required enantiomer of 4-hydroxy cyclopent-2-enyl acetate. Yields of over 80% are described, however in the best case this process uses whole cells at a ratio of 1 part cell biomass to 1 part substrate and the concentrations of product in the biotransformation are less than 1 %. Thus as well as the problems of product extraction from aqueous biological solutions, which are known to sometimes cause emulsion problems, the volumetric yields are low. It would be unlikely that the process as described would be suitable for large scale production in a chemical facility.

A second approach, in which an enzyme is used to selectively acetylate cis-cyclopent-1-ene-3,5-diol ('cis-diol'), has also been well described. This material can readily be prepared from cis-3,5-diacetoxy cyclopent-1-ene by simple saponification. One of the problems with this approach is that the enzymes that have been identified to date all have low selectivity in forming (1S, 4R)-(-)-4-hydroxy cyclopent-2-enyl acetate from the cis diol, with the (4S, 1R) enantiomer also being formed in significant quantities. However further esterification of the (4S, 1R)/(1S, 4R)-4-hydroxy cyclopent-2-enyl acetate mixture by the enzymes is much more selective and therefore high ee product can be obtained as a mixture with cis-3,5-diacetoxy cyclopent-1-ene as a by-product. In the described literature to date, this mixture is separated using chromatography. The cis-3,5-diacetoxy cyclopent-1-ene by-product may then clearly be recycled. The group of Theil has worked extensively with pancreatin/porcine pancreatic lipase, but has also screened enzymes from Mucor sp. (in house strain), Pseudomonas sp. (Amano PS), Lipozyme (Mucor miehi) and Candida sp. (382) for this biotransformation (Theil F. et al., Tetrahedron, 47, pp7569-82, (1991); DD 264707, DD 290663, DD 293136, DD 290662). Good results for the biotransformation with Mucor sp. were obtained at 94% ee and 85% yield, however this biocatalyst material is not readily available commercially and high concentrations of enzyme were required to get good reaction rates. For instance in the reaction described a 5-fold mass excess of enzyme preparation to starting cis-cyclopent-1-ene-3,5-diol was required in order to complete the reaction in 4 hours. Jommi G. et al. (Gazetta Chimica, Italiana, 118 , pp 863-864, (1988)) and Ghorpade SR. et al. (Tetrahedron Asymmetry, 10, pp 891-899, (1999)) have also described routes to (1S, 4R)-(-)-4-hydroxy cyclopent-2-enyl acetate using lipases in a similar transesterification reaction, however in the former case yields were low, whereas in the latter, very high enzyme to substrate ratios were required which would present poor economics. Johnson CR et al. (Tetrahedron Letters, 33 (48), pp 7287-7290, (1992)) described a biotransformation using Candida antartica (type B) enzyme where, after isolation, they obtained product (99% ee) in 48% yield from a 3 day biotransformation.

From the above it is apparent that although significant work and publications have appeared describing methods for the preparation of this important synthon, there are still obstacles to instituting an economic industrial process for its production. Either yields are poor, enzyme requirements are high or not readily available, or the product titres are low. In addition, the use of chromatography at large scale for separation of the (1S, 4R)-(-)- 4-hydroxy cyclopent-2-enyl acetate product from any formed cis-3,5-diacetoxy cyclopent-1-ene, while potentially giving good recoveries over the step, is expensive and not attractive at large scale. It is the object of the current invention to provide a process for the preparation of the (1S, 4R)-(-)- 4-hydroxy cyclopent-2-enyl esters, especially (1S, 4R)-(-)-4-hydroxy cyclopent-2-enyl acetate and propionate , which is both economic and readily scalable in a chemical facility and overcomes the obstacles of the known processes.

The instant invention solves this object and refers to a process for the preparation of (1S, 4R)- 4-hydroxy cyclopent-2-enyl esters comprising the steps of:
(a) reacting a cis-cyclopent-1-ene-3,5-diol, or racemic or a partially resolved 4-hydroxy cyclopent-2-enyl ester, with a suitable ester donor in the presence of a lipase from Alcaligenes sp.
   and
(b) recovering and purifying the produced (1S, 4R)-4-hydroxy cyclopent-2-enyl ester.

Surprisingly it has been found that that one of the best commercially available lipase enzyme preparations for carrying the transesterification of cis-cyclopent-1-ene-3,5-diol to produce (1S,4R)-(-)-4-hydroxy cyclopent-2-enyl acetate or -proprionate and its derivatives with both good yields and high ee is that produced by Alcaligenes sp. Such preparations can readily be obtained from Meito Sangyo, Japan, for instance Lipase QL, or the immobilised equivalents Lipase QLC and Lipase QLG, or alternatively from Roche Molecular Biochemicals, (D-68305 Mannheim Germany) eg product Chirazyme® L-10 or through traders such as Europa Bioproducts Ltd., (Europa House, 15-17 North Street, Wicken, Ely, Cambridge, CB7 5XW, UK.) eg EU032.

For the instant invention the lipase can be employed either in the free form or immobilized on a carrier material, such as for instance ceramic particles or diatomaceous earth to name only a few. Also, alternative formulations of this enzyme obtained for instance by purification, additive additions, recombinant expression of the Alcaligenes sp. selective lipase or modifications to the fermentation media, may be employed.

As described for other enzymes, the lipase from this species has relatively poor selectivity in the transesterification of the starting cis-diol and forms the required (1 S,4R) enantiomer at a ratio of approximately 4:1 compared to the (1 R,4S)-4-hydroxy cyclopent-2-enyl acetate. However, as the biotransformation proceeds the enzyme is highly selective in further esterifying the unwanted enantiomer to form cis-3,5-diacetoxy cyclopent-1-ene. This high selectivity is important in minimising the further esterification of the required (1 S,4R) enantiomer and loss of yield. As well as a good selectivity, this enzyme has good activity under the biotransformation conditions and can form product at high volumetric concentrations.

Although a range of solvent systems can be used with the enzyme, as described in the above literature, it is found that optimal selectivity is found when using low molecular weight ketones as the solvent - such as acetone, isobutylmethylketone or methylethyl ketone - combined with vinyl acetate or -propionate as the ester donor. The biotransformation may be carried out at a range temperatures as recommended by the manufacturer, however it is found that improved selectivity can be obtained at lower temperatures such as 10°C to roomtemperature, preferably 10-15°C, without resulting in excessive increase in enzyme useage.

Since the biotransformation is highly selective in the resolution of (1 S,4R)/(1 R,4S)-4-hydroxy cyclopent-2-enyl ester mixtures it is clearly advantageous to run such biotransformations until a high ee is reached, for instance >90% or more preferably >95% ee, so as to minimise losses on crystalisation.

Following biotransformation the enzyme can very readily be removed by filtration. In this respect addition of a filter aid, such as Celite, may facilitate removal, alternatively an immobilised enzyme preparation such as Lipase QLG or Lipase QLC may be used.

After stripping the solvent, the residual oil typically contains about 20-30% cis-3,5-diacetoxy cyclopent-1-ene by GC area percent. Although this contaminant can be removed by several re-crystalisations, this results in poor yields. However resuspension of the oil in an aqueous solution followed by extraction into an alkane solvent such as heptane results in good preferential removal of the cis-3,5-diacetoxy cyclopent-1-ene.

By altering the ionic strength of the aqueous solution the proportion of cis-3,5-diacetoxy cyclopent-1-ene and product of (1S,4R)-4-hydroxy cyclopent-2-enyl acetate partitioning into the heptane phase can be optimised. Back extraction of the product from the aqueous phase is readily accomplished by extraction into dichloromethane (or other suitable solvent). In a preferred instance of the invention the product containing oil is resuspended in 0.2-0.3M monosodium citrate and the cis-3,5-diacetoxy cyclopent-1-ene removed via extraction into an equal volume of heptane. For good extraction of the cis-3,5-diacetoxy cyclopent-1-ene it is found that about 3 extractions are required, but more or less may be used depending on the optimisation. The product is then isolated from the aqueous by extraction into an equal volume of dichloromethane. For good extraction, about 3 extractions are required, however more or less extractions may be used. As well as being useful for the removal of cis-3,5-diacetoxy cyclopent-1-ene, the aqueous extraction step is useful in removing any enzyme that might carry through from the the biotransformation/filtration. In this regard it is expected that a lowered pH as provided by the citrate solution used would also stop the hydrolytic activity of any surviving lipase in solution. Such activity could reduce ee and yield of the product during this extraction step.

Following extraction into dichloromethane, or similar suitable solvent, the product is very readily isolated by stripping the solvent and crystalising from an ether or ether:alkane mixture as has already been described, for instance a di-iso-propyl ether: heptane-mixture.

### Examples

### 1. Comparision of Alcaligenes sp. lipase and Immobilised Candida antarctica lipase

Lipase from Candida antactica, immobilised (obtained from Europa Bioproducts #EU085), and Alcaligenes sp (Europa Bioproducts - #EU032, equivalent to Meito Sangyo Lipase QL) were tested for their asymmetric transesterification of cis-cyclopent-1-ene-3,5-diol ('cis-diol') using vinyl acetate.

The reactions were all carried out in a vial (approx 5 ml volume) on a shaker (260-280 rpm) at room temperature. Quantities used and results are shown in Table 1 after 4 hours biotransformation. Product % is calculated according to GC area %, and ee's are determined by separation of the 2 enantiomers by direct injection onto a Cyclodextrin B chiral column - Supelco Beta Dex 120, length 30m, id 0.25mm, initial temperature 80°C for 10 minutes, ramp rate 5°C/minute, final temperature 160°C for 16 minutes. Retention time of (1R, 4S)-4-hydroxy cyclopent-2-enyl acetate is 28.6 minutes and of the product, (1S, 4R)-4-hydroxy cyclopent-2-enyl acetate is 28.9 minutes.

**Table 1: Comparision of Alcaligenes sp lipase and immobilised Candida antarctica lipase and use of different solvents.**

| Enzyme | cis-Diol | Solvent | Vinyl acetate | 4-hydroxy cyclopent-2-enyl acetate (%) | cis-3,5-diacetoxy cyclopent-1-ene (%) | ee (%) |
|---|---|---|---|---|---|---|
| EU085 - 2.5 mg | 26 mg | Acetonitrile - 0.5 ml | 0.075 ml | 58 | 36 | 87 |
| EU032 - 6.9 mg | 66 mg | Acetonitrile - 0.4 ml | 0.15 ml | 59 | 51 | 100 |
| EU032 - 6.5 mg | 66 mg | Acetone - 0.4 ml | 0.15 ml | 66 | 34 | 100 |
| EU032 - 6.6 mg | 65 mg | Tetrahydro furan (THF) - 0.4 ml | 0.15 ml | 52 | 48 | 100 |

### 2. Further testing of Alcaligenes sp lipase with different solvents.

Using a similar method to that described in Example 1 various solvents were tested in a biotransformation at room temperature. Results provided in Table 2.

**Table 2: Conversion of cis-cyclopent-1-ene-3,5-diol to 4-hydroxy cyclopent-2-enyl acetate by Alcaligenes sp. lipase at room temperature with various solvents after 90 minutes**

| Enzyme | cis-Diol | Solvent | Vinyl acetate | 4-hydroxy cyclopent-2-enyl acetate (%) | cis-3,5-diacetoxy cyclo-pent-1-ene (%) | ee (%) |
|---|---|---|---|---|---|---|
| EU032 - 4.5 mg | 65 mg | Acetone - 0.4 ml | 0.13 ml | 72 | 28 | 90 |
| EU032 - 5.3 mg | 61 mg | Ethyl methyl ketone - 0.4 ml | 0.13 ml | 65 | 35 | 96 |
| EU032 - 4.7 mg | 64 mg | Isobutyl methyl ketone - 0.4 ml | 0.13 ml | 57 | 43 | 98 |
| EU032 - 4.36 mg | 59 mg | Ethyl acetate - 0.4 ml | 0.13 ml | 65 | 35 | 87 |

### 3. Use of immobilised preparations of of Alcaligenes sp lipase.

Using a similar method to that described in Example 1, 2 commercially available immobilised Alcaligenes sp lipase preparations were tested. Lipase QLC is the lipase immobilised on ceramic particles and Lipase QLG is the lipase immobilised on a diatomaceous earth. Results provided in Table 3.

**Table 3: Conversion of cis-cyclopent-1-ene-3,5-diol to 4-hydroxy cyclopent-2-enyl acetate immobilise lipase preparations**

| Enzyme | cis-Diol | Solvent | Vinyl acetate | 4-hydroxy cyclopent-2-enyl acetate (%) | cis-3,5-diacetoxy cyclopent-1-ene (%) | ee (%) |
|---|---|---|---|---|---|---|
| Lipase QLC - 30 mg | 59 mg | Acetone - 0.4 ml | 0.12 ml | 68 | 32 | >99 |
| Lipase QLG - 28 mg | 58mg | Acetone - 0.4 ml | 0.12 ml | 69 | 31 | >99 |

| | | | | | | |
|---|---|---|---|---|---|---|
| Reaction time 2 hours at room temperature | | | | | | |

### 4. Biotransformation at larger scale using Alcaligenes sp. lipase.

The following biotransformation was set-up in a stirrer 800 ml water jacketed glass vessel.
Lipase QL - 1.81 g
cis-cyclopent-1-ene-3,5-diol - 36 g (containing a small amount of isomers)
Vinyl acetate - 68 ml
Acetone - 230 ml

### Temperature 13.5°C

After 345 minutes slow stirring to ensure good suspension of the powder, GC analysis showed:
(1 S, 4R)-4-hydroxy cyclopent-2-enyl acetate at 75% GC area % (3.5% of this other isomers), with an ee of 96%, and the by-product,
cis-3,5-diacetoxy cyclopent-1-ene - 25%.

### 5. Separation of (1S, 4R)-4-hydroxy cyclopent-2-enyl acetate from cis-3,5-diacetoxy cyclopent-1-ene

Material from the biotransformation in Example 4. was stripped and taken up in 200 ml 0.2 M monosodium citrate (at room temperature) which had been adjusted to pH 4.1 by the addition of a small amount of 4M NaOH. The solution was then extracted with 200 ml heptane, 3 times, after which the aqueous phase was extracted with 200 ml dichloromethane 3 times. Analysis of the heptane and dichloromethane phases showed:
Heptane: 4-hydroxy cyclopent-2-enyl acetate - 6 % (GC area)
cis-3,5-diacetoxy cyclopent-1-ene - 94 %.
Dichloromethane: 4-hydroxy cyclopent-2-enyl acetate - 92 % (GC area) (including isomers)
cis-3,5-diacetoxy cyclopent-1-ene - 5 %.

Stripping of the dichloromethane and crystalising from a binary solvent system of 1 part diisopropyl ether to 0.4 parts heptane yielded (1S, 4R)-(-)-4-hydroxy cyclopent-2-enyl acetate crystals at >99% ee by chiral GC and >99% purity. Measurement of the optical rotation of a 1% solution of the material in chloroform gave a value of -68.5° (λ=589nm).

### 6. Preparation of (1S, 4R)-4-hydroxy cyclopent-2-enyl acetate from cis-cyclopent-1-ene-3,5-diol

A biotransformation was set-up using the following conditions:
Lipase QL - 300mg
cis-cyclopent-1-ene-3,5-diol - 5.53 g (98.7% pure, GC)
Vinyl acetate - 10 ml
Acetone - 35 ml

With stirring at temperature 10°C. After 6 hours, 1.4 g Celite Hyflo was added and the solution stirred for a further 5 minutes. The solution was then filtered and the solvent stripped. To the residual oil was added 35 ml of 0.25M monosodium citrate. This solution was extracted with 35 ml heptane a total of 3 times. The product was then extracted from the aqueous phase into 35 ml dichloromethane, a total of 3 times. After each organic extraction the phase was back washed with a small (1 ml) water to minimise salt carry through. The product was then crystalised to give a total of 4.93 g (1S, 4R)-4-hydroxy cyclopent-2-enyl acetate at 98% purity and >96% ee (yield on starter 63%). Analysis also showed that about 90% of the product was recovered in the dichloromethane phase whereas about 80% of the cis-3,5-diacetoxy cyclopent-1-ene was removed into the heptane phase.

### 7. Preparation of (1S, 4R)-4-hydroxy cyclopent-2-enyl propionate.

By using vinyl propionate instead of vinyl acetate it is possible to produce (1S, 4R)-4-hydroxy-cyclopent-2-enyl propionate instead of (1S, 4R)-4-hydroxy cyclopent-2-enyl acetate using a similar methodology and enzyme to that described in the earlier examples. The following reaction was set up in a vial with shaking at room temperature:

| | |
|---|---|
| cis-cyclopent-1-ene-3,5-diol | 65 mg |
| Lipase QL | 4.9 mg |
| Acetone | 0.4 ml |
| Vinyl propionate | 0.14 ml |

Analysis by GC of samples taken at various times gave the following results

| Time (min) | cis-cyclopent-1-ene-3,5-diol (GC % area) | 4-hydroxy-cyclopent-2-enyl propionate (GC % area) | Propionic acid 4-propionyloxy-cyclopent-2-enyl ester (GC %area) | ee (%) |
|---|---|---|---|---|
| 90 | 13 | 69 | 18 | 66 |
| 180 | 1 | 65 | 34 | 95 |
| 230 | 0 | 62 | 38 | 97 |

After 230 minutes, the enzyme was removed by centrifugation and the solvent stripped. To the resulting oil was added 0.5 ml 0.25M monosodium citrate. The propionic acid 4-propionyloxy-cyclopent-2-enyl ester was then removed by extraction into 0.5 ml heptane. The 4-hydroxy-cyclopent-2-enyl propionate was isolated from the aqueous by extraction into methyl tert-butyl ether. This was then stripped from the oil to leave a product containing 4-hydroxy-cyclopent-2-enyl propionate 96 %, propionic acid 4-propionyloxy-cyclopent-2-enyl ester 4% (by GC area %). Measurement of the optical rotation of this material at c=0.9, in chloroform gave a value of -46°. This compares to a value of -56.7° at c=1, chloroform reported by Theil F et al (Theil F et al., Liebs Ann. Chem., pp195-200, (1991)) for (1S, 4R)-4-hydroxy cyclopent-2-enyl propionate, confirming the configuration.

## Claims

1. Process for the preparation of (1S, 4R)-4-hydroxy cyclopent-2-enyl esters comprising the steps of:
a) reacting a cis-cyclopent-1-ene-3,5-diol, or racemic or a partially resolved 4-hydroxy cyclopent-2-enyl ester, with a suitable ester donor in the presence of a lipase from Alcaligenes sp. and
b) recovering and purifying the produced (1S, 4R)-4-hydroxy cyclopent-2-enyl ester.

2. Process according to claim 1, wherein the reaction is carried out at a temperature in the range from 10°C to 15°C.

3. Process according to claim 1 or 2, wherein the Alcaligenes sp lipase is used in the free form or immobilized on a carrier material.

4. Process according to at least one of the claims 1 to 3, wherein the reaction is conducted in a low molecular weight ketone as a solvent.

5. Process according to claim 4, wherein acetone, isobutylmethylketone or methylethylketone are used as solvents.

6. Process according to at least one of the claims 1 to 5, wherein vinyl acetate or vinyl propionate are used as an ester donor.

7. Process according to at least one of the claims 1 to 6, wherein the biotransformation is run to an ee > 95%.

8. Process according to at least one of the claims 1 to 7, wherein the (1S, 4R)-4-hydroxy cyclopent-2-enyl ester is recovered by filtration of the biocatalyst, the solvent is being stripped off and the residual oil is resuspended in an aqueous solution, the cis-3,5-diacetoxy cyclopent-1-ene is removed via extraction into an alkane solvent.

## Patentansprüche

1. Verfahren zur Herstellung von (1S,4R)-4-Hydroxy-cyclopent-2-enylestern, welches die folgenden Schritte umfaßt:
a) Umsetzung eines cis-Cyclopent-1-en-3,5-diols oder eines racemischen oder teilweise racematgespaltenen 4-Hydroxycyclopent-2-enylesters mit einem geeigneten Esterdonor in Gegenwart einer Lipase von Alcaligenes sp. und
b) die Isolierung und Aufreinigung des produzierten (1S,4R)-4-Hydroxycyclopent-2-enylesters.

2. Verfahren nach Anspruch 1, wobei die Umsetzung bei einer Temperatur im Bereich von 10°C bis 15°C durchgeführt wird.

3. Verfahren nach Anspruch 1 oder 2, wobei die Alcaligenes-sp.-Lipase in freier Form oder immobilisiert auf einem Trägermaterial verwendet wird.

4. Verfahren nach wenigstens einem der Ansprüche 1 bis 3, wobei die Umsetzung in einem niedermolekularen Keton als Lösungsmittel durchgeführt wird.

5. Verfahren nach Anspruch 4, wobei als Lösungsmittel Aceton, Isobutylmethylketon oder Methylethylketon verwendet werden.

6. Verfahren nach wenigstens einem der Ansprüche 1 bis 5, wobei als Esterdonor Essigsäurevinylester oder Propionsäurevinylester verwendet wird.

7. Verfahren nach wenigstens einem der Ansprüche 1 bis 6, wobei die biologische Umwandlung bis zu einem ee > 95% durchgeführt wird.

8. Verfahren nach wenigstens einem der Ansprüche 1 bis 7, wobei der (1S,4R)-4-Hydroxycyclopent-2-enylester durch Filtrieren des biologischen Katalysators, Abstreifen des Lösungsmittels und Resuspendieren des verbliebenen Öls in einer wäßrigen Lösung isoliert wird, wobei das cis-3,5-Diacetoxycyclopent-1-en durch Extraktion in ein Alkan-Lösungsmittel entfernt wird.

## Revendications

1. Procédé pour la préparation d'esters de (1S,4R)-4-hydroxycyclopent-2-ényle, comprenant les étapes de :
a) mise en réaction d'un *cis*-cyclopent-1-éne-3,5-diol ou d'un ester de 4-hydroxycyclopent-2-ényle racémique ou partiellement résolu, avec un donneur d'ester approprié, en présence d'une lipase d*'Alcaligenes sp* et
b) récupération et purification de l'ester de (1S,4R)-4-hydroxycyclopent-2-ényle produit.

2. Procédé selon la revendication 1, dans lequel la réaction est effectuée à une température dans la plage allant de 10 °C à 15 °C.

3. Procédé selon la revendication 1 ou 2, dans lequel la lipase d'*Alcaligenes sp*. est utilisée sous la forme libre ou immobilisée sur une matière de support.

4. Procédé selon au moins l'une des revendications 1 à 3, dans lequel la réaction est effectuée dans une cétone de faible masse moléculaire en tant que solvant.

5. Procédé selon la revendication 4, dans lequel on utilise comme solvants l'acétone, l'isobutylméthylcétone ou la méthyléthylcétone.

6. Procédé selon au moins l'une des revendications 1 à 5, dans lequel on utilise comme donneur d'ester l'acétate de vinyle ou le propionate de vinyle.

7. Procédé selon au moins l'une des revendications 1 à 6, dans lequel la biotransformation est effectuée jusqu'à un excès d'énantiomère ee > 95 %.

8. Procédé selon au moins l'une des revendications 1 à 7, dans lequel l'ester de (1S,4R)-4-hydroxycyclopent-2-ényle est récupéré par séparation du biocatalyseur par filtration, le solvant est éliminé et l'huile résiduelle est remise en suspension dans une solution aqueuse, le *cis*-3,5-diacétocyclopent-1-ène est éliminé par extraction dans un solvant de type alcane.
